# EUROPÄISCHE PATENTANMELDUNG

(11) **EP 2 869 227 A1**
(43) Veröffentlichungstag der Anmeldung: **06.05.2015**
(21) Anmeldenummer: 14151848.0
(22) Anmeldetag: 21.01.2014
(51) Int. Cl.: G06F 19/00

(54) **Kontrollsystem**

(30) Priorität: 30.10.2013 DE 102013111964
(71) Anmelder: Eckardt, Manuel, 35781 Weilburg (DE)
(72) Erfinder: Eckardt, Manuel, 35781 Weilburg (DE)
(74) Vertreter: Ruckh, Rainer Gerhard

(57) **Zusammenfassung**

Die Erfindung betrifft ein Kontrollsystem (1) mit einer einen Bildschirm (6) aufweisenden Rechnereinheit, in welcher ein Formular abgespeichert ist, welches einem Benutzer während einer Präsentationszeit am Bildschirm (6) angezeigt wird. Der Rechnereinheit sind ein Benutzeridentifikationssystem und ein Sensor zugeordnet, wobei der Sensor dazu ausgebildet ist, zu erfassen, ob ein Benutzer das am Bildschirm (6) angezeigte Formular liest. Mittels des Benutzeridentifikationssystems wird eine den Benutzer eindeutig kennzeichnende Benutzer-Identifikation erfasst. In der Rechnereinheit wird eine positive Kontrollmeldung, dass der Benutzer den Inhalt des Formulars zur Kenntnis genommen und/oder akzeptiert hat, nur dann generiert, wenn vom Sensor generierte Signale signalisieren, dass der Benutzer das Formular während der Präsentationszeit ununterbrochen gelesen hat. Der Benutzer ist anhand der Eingabe einer Benutzer-Identifikation im Benutzeridentifikationssystem eindeutig identifiziert.

## Beschreibung

Die Erfindung betrifft ein Kontrollsystem gemäß dem Oberbegriff des Anspruchs 1.

Ein derartiges Kontrollsystem dient generell zur rechnergesteuerten Bereitstellung von Formularen. Derartige Formulare können insbesondere von Patientenaufklärungsbögen gebildet sein.

Mit einem derartigen Patientenaufklärungsbogen wird ein Patient über eine an ihm durchzuführende Behandlung oder Operation aufgeklärt. Insbesondere wird im Patientenaufklärungsbogen auf Risiken und Gefahren, die mit der Behandlung oder Operation verbunden sind, hingewiesen.

Diese Patientenaufklärungsbögen können in bekannter Weise auf Rechnereinheiten gespeichert werden, so dass in bekannter Weise eine rechnergesteuerte Dokumentenverwaltung dieser Patientenaufklärungsbögen erfolgen kann.

Die wesentliche Funktion eines Patientenaufklärungsbogens besteht darin, dass ein zuständiger Arzt gegenüber seinem Patienten juristisch abgespeichert dokumentiert, dass er den Patienten über alle Risiken und Gefahren einer Behandlung oder Operation aufgeklärt hat.

Um das zu bewerkstelligen, erfolgt ein Arzt-Patienten-Gespräch. In diesem Gespräch liest der Arzt im Wesentlichen den Inhalt des Patientenaufklärungsbogens vor, wobei bei Nachfragen durch den Patienten der Arzt hierzu nähere Erläuterungen gibt. Als Quittung und juristische Absicherung für den Arzt dokumentiert dann der Patient, dass er über den Inhalt des Patientenaufklärungsbogens informiert wurde und sich mit der Behandlung oder Operation einverstanden erklärt.

Diese Vorgehensweise ist für den Arzt jedoch äußerst zeitaufwändig, da er den gleichartigen Aufklärungsvorgang mit einem Patientenaufklärungsbogen für jeden Patienten in einem separaten Arzt-Patienten-Gespräch, in welchem er den gesamten Patientenaufklärungsbogen erläutern muss, durchführen muss.

Der Erfindung liegt die Aufgabe zugrunde, ein Kontrollsystem bereitzustellen, mittels derer eine automatisierte, selbsttätige Überwachung des Lesens von Formularen ermöglicht wird.

Zur Lösung dieser Aufgabe sind die Merkmale des Anspruchs 1 vorgesehen. Vorteilhafte Ausführungsformen und zweckmäßige Weiterbildungen der Erfindung sind in den Unteransprüchen beschrieben.

Die Erfindung betrifft ein Kontrollsystem mit einer einen Bildschirm aufweisenden Rechnereinheit, in welcher ein Formular abgespeichert ist, welches einem Benutzer während einer Präsentationszeit am Bildschirm angezeigt wird. Der Rechnereinheit sind ein Benutzeridentifikationssystem und ein Sensor zugeordnet, wobei der Sensor dazu ausgebildet ist, zu erfassen, ob ein Benutzer das am Bildschirm angezeigte Formular liest. Mittels des Benutzeridentifikationssystems wird eine den Benutzer eindeutig kennzeichnende Benutzer-Identifikation erfasst. In der Rechnereinheit wird eine positive Kontrollmeldung, dass der Benutzer den Inhalt des Formulars zur Kenntnis genommen und/oder akzeptiert hat, nur dann generiert, wenn vom Sensor generierte Signale signalisieren, dass der Benutzer das Formular während der Präsentationszeit ununterbrochen gelesen hat. Der Benutzer ist anhand der Eingabe einer Benutzer-Identifikation im Benutzeridentifikationssystem eindeutig identifiziert.

Mit dem erfindungsgemäßen Kontrollsystem kann somit einerseits eindeutig erfasst werden, wer ein bestimmtes Formular gelesen hat und weiterhin, ob der eindeutig identifizierte Benutzer während einer Präsentationszeit das Formular tatsächlich ununterbrochen im Blick gehabt hat, wodurch kontrolliert wird, ob der Benutzer das Formular tatsächlich ununterbrochen gelesen hat.

Damit kann mit hoher Sicherheit, insbesondere juristisch abgespeichert, erfasst werden, ob dem Benutzer der Inhalt des Formulars zur Kenntnis gebracht wurde, beziehungsweise ob der Inhalt des Formulars vom Benutzer akzeptiert wurde.

Das Kontrollsystem vereinfacht und automatisiert somit die Dokumentation des Zur-Kenntnis-Bringens eines Formulars, das heißt das bislang erforderliche persönliche Gespräch zwischen einem Benutzer und einem Dritten, in welchem dem Benutzer durch Vorlesen des Formulars dessen Inhalt zur Kenntnis gebracht wird, kann vollständig oder zumindest weitgehend entfallen.

Das erfindungsgemäße Kontrollsystem kann allgemein für Formulare in den unterschiedlichsten Anwendungsfällen, wie zum Beispiel Formularen in Ämtern oder aber auch bei Polizeikontrollen eingesetzt werden.

Besonders vorteilhaft sind die Formulare von Patientenaufklärungsbögen gebildet, auf welche im Folgenden ohne Beschränkung der Allgemeinheit ausschließlich Bezug genommen wird.

Durch den Einsatz des erfindungsgemäßen Kontrollsystems können Arzt-Patienten-Gespräche, die bislang für jeden Patientenaufklärungsbogen obligatorisch waren und in welcher jeweils der gesamte Patientenaufklärungsbogen vom Arzt vorgelesen werden musste, auf ein Geringstmaß reduziert werden, wodurch eine erhebliche Zeitersparnis für die behandelnden Ärzte erzielt wird.

Ein wesentlicher Aspekt der Erfindung besteht darin, dass mit dem Sensor des Kontrollsystems fortlaufend erfasst wird, ob ein Benutzer seinen Blick auf den Patientenaufklärungsbogen richtet, das heißt ob er diesen während einer Präsentationszeit liest.

Wird mit dem Sensor festgestellt, dass der Benutzer seinen Blick nicht fortwährend auf den am Bildschirm angezeigten Patientenaufklärungsbogen richtet, wird anhand der vom Sensor generierten Signale in der Rechnereinheit sofort der Lesevorgang beendet und eine negative Kontrollmeldung generiert, dass der Lesevorgang als nicht erfolgreich abgebrochen wurde. Damit wird verhindert, dass der Benutzer durch Eingabe seiner Benutzer-Identifikation in das Benutzeridentifikationssystem den Lesevorgang noch als gültig deklarieren kann.

Die Quittierung eines Lesevorgangs als gültig durch Eingabe der Benutzer-Identifikation in das Benutzeridentifikationssystem kann somit nur nach einem Lesevorgang erfolgen, der mit dem Sensor als vollständig erkannt wurde.

Im einfachsten Fall könnte auf diese Weise ein Arzt-Patienten-Gespräch vollständig ersetzt werden. In diesem Fall würde ein Benutzer mit der Eingabe seiner Benutzer-Identifikation stets dokumentieren, dass er den Inhalt des Patientenaufklärungsbogens verstanden und akzeptiert hat.

Gemäß einer besonders vorteilhaften Ausgestaltung der Erfindung weist die Rechnereinheit eine Ein- /Ausgabeeinheit mit mehreren Eingabefeldern auf, die dem Benutzer eine Differenzierung bei der Quittierung des Lesevorgangs des Patientenaufklärungsbogens ermöglichen.

So kann der Benutzer nach erfolgreichem Lesen des Patientenaufklärungsbogens durch Betätigen eines ersten Eingabefelds dokumentieren, dass er den Inhalt des Patientenaufklärungsbogens verstanden und akzeptiert hat. Alternativ kann der Benutzer durch Betätigen eines zweiten Eingabefelds dokumentieren, dass er den Patientenaufklärungsbogen zwar gelesen hat, jedoch noch Fragen hierzu hat.

Im ersten Fall ist kein Arzt-Patienten-Gespräch mehr notwendig, da der Benutzer, das heißt Patient, sein Einverständnis mit der im Patientenaufklärungsbogen erläuterten Behandlung oder Operation gegeben hat. Nur im zweiten Fall wird ein Arzt-Patienten-Gespräch notwendig, da hier der Patient mit seiner Betätigung des zweiten Eingabefelds signalisiert, dass er noch Beratungsbedarf hat.

Durch diese Differenzierung wird eine erhebliche Erleichterung und Zeitersparnis für den behandelnden Arzt erzielt, der er nur mit den Patienten ein Arzt-Patienten-Gespräch führen muss, die tatsächlich Beratungsbedarf haben.

Die Rechnereinheit des erfindungsgemäßen Kontrollsystems ist vorteilhaft von einem Tablet-PC gebildet.

Besonders vorteilhaft sind der Sensor und das Benutzeridentifikationssystem in der Rechnereinheit integriert. Die Rechnereinheit weist einen Prozessor auf, in welchem die vom Sensor und Benutzeridentifikationssystem generierten Signale ausgewertet werden.

Das erfindungsgemäße Kontrollsystem weist damit einen einfachen Aufbau auf, wobei besonders vorteilhaft ist, dass das Kontrollsystem im Wesentlichen aus handelsüblichen Rechner-Komponenten besteht.

Besonders vorteilhaft ist der Sensor ein optischer Sensor, und weist eine Kamera auf.

Mit diesem Kamerasystem kann genau erfasst werden, ob ein Benutzer seinen Blick auf den Bildschirm der Rechnereinheit richtet.

Weiter vorteilhaft ist das Benutzeridentifikationssystem ein Fingerprintsystem.

Ein derartiges Fingerprintsystem ist äußerst leicht zu bedienen und erlaubt zudem eine eindeutige Identifizierung von Benutzern.

Die Erfindung wird im Folgenden anhand der Zeichnungen erläutert. Es zeigen:
- Figur 1:: Schematische Darstellung eines Ausführungsbeispiels des erfindungsgemäßen Kontrollsystems.
- Figur 2:: Blockschaltbild von Komponenten des Kontrollsystems gemäß Figur 1.

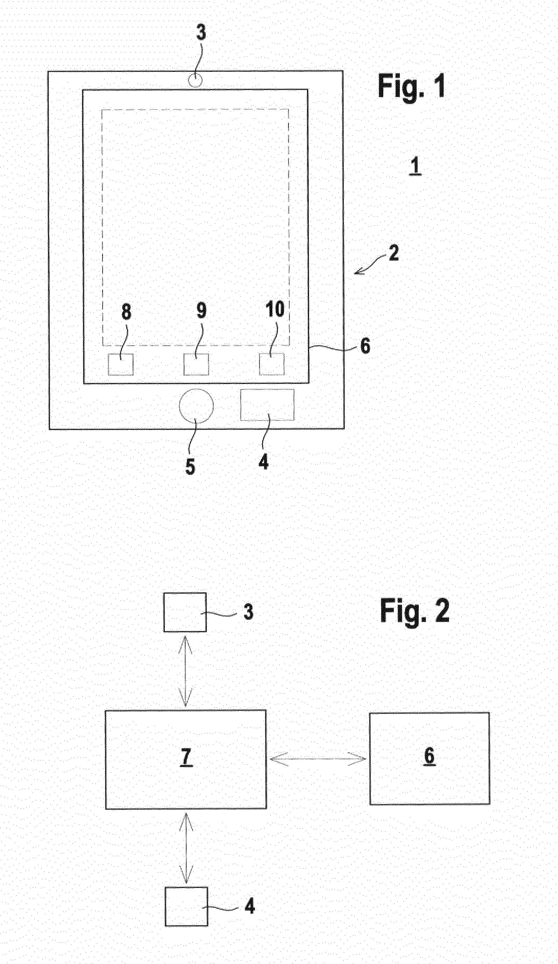

Figur 1 zeigt schematisch eine Ausführungsform des erfindungsgemäßen Kontrollsystems 1. Das Kontrollsystem 1 besteht im vorliegenden Fall aus einem Tablet-PC 2, der als zusätzliche Komponenten einen optischen Sensor 3 mit einer Kamera und als Benutzeridentifikationssystem ein Fingerprintsystem 4 aufweist.

Weiterhin weist der Tablet-PC 2 einen Bedienknopf 5 sowie einen Bildschirm 6 auf, wobei der Bildschirm 6 in bekannter Weise als Touch-Screen ausgebildet ist.

Wie Figur 2 zeigt, weist der Tablet-PC 2 einen Prozessor 7 auf, an welchen der optische Sensor 3 und das Fingerprintsystem 4 angeschlossen ist, wobei der Tablet-PC 2 diese Einheiten ansteuert und von diesen generierte Signale auswertet. Weiterhin steuert der Prozessor 7 in bekannter Weise die Bildschirmfunktion des Tablet-PCs 2.

Im Prozessor 7 des Tablet-PCs 2 ist wenigstens ein Formular in Form eines Patientenaufklärungsbogens abgespeichert, der auf dem Bildschirm 6 angezeigt werden kann.

Auf dem Bildschirm 6 sind weiter als Elemente einer Ein-/ Ausgabeeinheit diverse Bedienfelder vorhanden, mittels derer ein Benutzer spezifische Eingaben vornehmen kann. Von diesen Bedienfeldern sind in Figur 1 ein Aktivierungsfeld 8, ein erstes Eingabefeld 9 und ein zweites Eingabefeld 10 dargestellt.

Der Patientenaufklärungsbogen enthält in bekannter Weise Informationen über eine durchzuführende Behandlung oder Operation durch einen Arzt. Insbesondere enthält der Patientenaufklärungsbogen eine Belehrung über die Gefahren und Risiken der Behandlung oder Operation. Seitens des Arztes muss der jeweilige Patientenaufklärungsbogen dem Patienten juristisch abgespeichert zur Kenntnis gebracht werden.

Hierzu dient das erfindungsgemäße Kontrollsystem 1.

Ein Patient, der einen Patientenaufklärungsbogen zur Kenntnis nehmen soll, verwendet hierzu, und zwar ohne Beisein des Arztes, den Tablet-PC 2.

Durch Betätigen des Aktivierungsfelds 8 lädt der Patient als Benutzer des Kontrollsystems 1 den Patientenaufklärungsbogen für eine Präsentationszeit auf den Bildschirm 6 des Tablet-PCs 2. Die Präsentationszeit startet mit dem Laden des Patientenaufklärungsbogens. Die Dauer der Präsentationszeit hängt von den nachfolgenden Aktionen des Benutzers ab. Optional kann im Prozessor 7 eine Zeitsteuerung vorgesehen sein, die die Dauer der Präsentationszeit überwacht.

Sobald der Patientenaufklärungsbogen auf den Bildschirm 6 geladen ist, wird mit dem optischen Sensor 3 überwacht, ob der Benutzer den Blick fortlaufend auf den Bildschirm 6 richtet, wodurch selbsttätig geprüft wird, ob der Benutzer den Patientenaufklärungsbogen liest.

Wird mit dem optischen Sensor 3 festgestellt, dass der Benutzer den Bildschirm 6 nur mit Unterbrechungen betrachtet, wird dies im Prozessor 7 als Nicht-Lesen des Patientenaufklärungsbogens gewertet. Darauf unterbricht der Prozessor 7 sofort die Anzeige des Patientenaufklärungsbogens und generiert eine negative Kontrollmeldung, dass der Lesevorgang durch den Benutzer als erfolglos abgebrochen wurde.

Zusätzlich kann auch die Zeitsteuerung im Prozessor 7 zur Kontrolle des Lesevorgangs unterbrochen werden. Hierzu ist vorteilhaft im Prozessor 7 eine Sollzeit abgespeichert. Betrachtet der Benutzer den Patientenaufklärungsbogen für eine Zeit, die unter der Sollzeit liegt, wird im Prozessor 7 ebenfalls eine negative Kontrollmeldung generiert. Damit wird verhindert, dass der Benutzer durch bloßes Betrachten des Bildschirms 6 ein Lesen des Patientenaufklärungsbogens vortäuscht.

Wird dagegen vom optischen Sensor 3 ein kontinuierliches Lesen des Patientenaufklärungsbogens für eine Zeit größer als die Sollzeit registriert, wird dies als gültiger Lesevorgang im Prozessor 7 gewertet.

In diesem Fall beendet der Benutzer die Präsentationszeit des Patientenaufklärungsbogens regulär durch Betätigen des ersten Eingabefelds 8 oder des zweiten Eingabefelds 10.

Durch Betätigen des entsprechend gekennzeichneten ersten Eingabefelds 9 bestätigt der Benutzer, dass der den Patientenaufklärungsbogen gelesen und verstanden hat, und dass er mit dem Inhalt des Patientenaufklärungsbogens einverstanden ist, das heißt er akzeptiert die im Patientenaufklärungsbogen vorgeschlagene Behandlung und Operation.

Vorteilhaft wird über den Prozessor 7 ein entsprechender Vermerk in den Patientenaufklärungsbogen eingeschrieben.

Anschließend quittiert der Benutzer mit Eingabe seines Fingerabdrucks als Benutzer-Identifikation den Patientenaufklärungsbogen. Damit ist der Benutzer eindeutig identifiziert und der gerade ausgefüllte Patientenaufklärungsbogen eindeutig zugeordnet.

In diesem Fall ist kein Arzt-Patienten-Gespräch erforderlich um den jeweiligen Patienten den Patientenaufklärungsbogen zu erläutern. Vielmehr erfolgte in diesem Fall die Aufklärung des Patienten völlig selbsttätig über das Kontrollsystem 1.

Bestätigt der Benutzer nach Lesen des Patientenaufklärungsbogens am Bildschirm 6 des Tablet-PCs 2 das zweite Eingabefeld 10, so gibt der Benutzer damit zu erkennen, dass er noch Fragen zum Patientenaufklärungsbogen hat. Vorteilhaft wird dann mittels des Prozessors 7 ein entsprechender Vermerk in den Patientenaufklärungsbogen eingeschrieben.

Anschließend gibt der Benutzer seine Benutzer-Identität ein, indem er einen Fingerabdruck im Fingerprintsystem 4 eingibt.

Auch in diesem Fall wird damit ein korrektes Lesen des Patientenaufklärungsbogens durch den Benutzer quittiert.

Durch den Vermerk im Patientenaufklärungsbogen, dass der Benutzer, das heißt der Patient, noch Fragen hat, wird dem zuständigen Arzt signalisiert, dass der Patient noch Beratungsbedarf hat. Daher folgt auf das automatisch mit dem Kontrollsystem 1 überwachte Lesen des Patientenaufklärungsbogens noch ein Arzt-Patienten-Gespräch, in welchem der Arzt die offenen Fragen des Patienten klären kann.

### Bezugszeichenliste

- (1): Kontrollsystem
- (2): Tablet-PC
- (3): Optischer Sensor
- (4): Fingerprintsystem
- (5): Bedienknopf
- (6): Bildschirm
- (7): Prozessor
- (8): Aktivierungsfeld
- (9): erstes Eingabefeld
- (10): zweites Eingabefeld

## Patentansprüche

1. Kontrollsystem (1) mit einer einen Bildschirm (6) aufweisenden Rechnereinheit, in welcher ein Formular abgespeichert ist, welches einem Benutzer während einer Präsentationszeit am Bildschirm (6) angezeigt wird, **dadurch gekennzeichnet, dass** der Rechnereinheit ein Benutzeridentifikationssystem und ein Sensor zugeordnet sind, wobei der Sensor dazu ausgebildet ist, zu erfassen, ob ein Benutzer das am Bildschirm (6) angezeigte Formular liest, und wobei mittels des Benutzeridentifikationssystems eine den Benutzer eindeutig kennzeichnende Benutzer-Identifikation erfasst wird, und dass in der Rechnereinheit eine positive Kontrollmeldung, dass der Benutzer den Inhalt des Formulars zur Kenntnis genommen und/oder akzeptiert hat, nur dann generiert wird, wenn vom Sensor generierte Signale signalisieren, dass der Benutzer das Formular während der Präsentationszeit ununterbrochen gelesen hat, und wenn der Benutzer anhand der Eingabe einer Benutzer-Identifikation im Benutzeridentifikationssystem eindeutig identifiziert ist.

2. Kontrollsystem nach Anspruch 1, **dadurch gekennzeichnet, dass** der Sensor und das Benutzeridentifikationssystem in der Rechnereinheit integriert sind.

3. Kontrollsystem nach einem der Ansprüche 1 oder 2, **dadurch gekennzeichnet, dass** die Rechnereinheit einen Prozessor aufweist, in welchem die vom Sensor und Benutzeridentifikationssystem generierten Signale ausgewertet werden.

4. Kontrollsystem nach einem der Ansprüche 1 bis 3, **dadurch gekennzeichnet, dass** die Rechnereinheit ein Tablet-PC (2) ist.

5. Kontrollsystem nach einem der Ansprüche 1 bis 4, **dadurch gekennzeichnet, dass** der Sensor ein optischer Sensor (3) ist.

6. Kontrollsystem nach Anspruch 5, **dadurch gekennzeichnet, dass** der optische Sensor (3) eine Kamera aufweist.

7. Kontrollsystem nach einem der Ansprüche 1 bis 6, **dadurch gekennzeichnet, dass** das Benutzeridentifikationssystem ein Fingerprintsystem (4) ist.

8. Kontrollsystem nach einem der Ansprüche 1 bis 7, **dadurch gekennzeichnet, dass** das Formular ein Patientenaufklärungsbogen ist.

9. Kontrollsystem nach einem der Ansprüche 1 bis 8, **dadurch gekennzeichnet, dass** die Rechnereinheit eine Ein-/ Ausgabeeinheit aufweist.

10. Kontrollsystem nach Anspruch 9, **dadurch gekennzeichnet, dass** die Ein-/ Ausgabeeinheit ein Aktivierungsfeld (8) aufweist, durch dessen Betätigung durch einen Benutzer die Anzeige des Formulars am Bildschirm (6) gestartet wird.

11. Kontrollsystem nach einem der Ansprüche 9 oder 10, **dadurch gekennzeichnet, dass** die Ein-/ Ausgabeeinheit ein erstes Eingabefeld (9) aufweist, durch dessen Betätigung ein Benutzer quittiert, dass er das Formular gelesen und akzeptiert hat.

12. Kontrollsystem nach einem der Ansprüche 1 bis 11, **dadurch gekennzeichnet, dass** die Ein-/ Ausgabeeinheit ein zweites Eingabefeld (10) aufweist, durch dessen Betätigung ein Benutzer quittiert, dass er das Formular gelesen hat und er noch Fragen hierzu hat.

13. Kontrollsystem nach einem der Ansprüche 11 oder 12, **dadurch gekennzeichnet, dass** durch Betätigen des zweiten oder dritten Eingabefelds (9, 10) die Präsentationszeit regulär beendet wird.

14. Kontrollsystem nach einem der Ansprüche 1 bis 13, **dadurch gekennzeichnet, dass** in der Rechnereinheit eine negative Kontrollmeldung, dass der Benutzer das Formular nicht gelesen hat, generiert wird, wenn mittels des Sensors festgestellt wird, dass der Benutzer das Lesen des Formulars unterbrochen hat.

15. Kontrollsystem nach Anspruch 14, **dadurch gekennzeichnet, dass** mit Generierung der negativen Kontrollmeldungen die Präsentationszeit vorzeitig und irregulär beendet wird.
